(19) Europäisches Patentamt
European Patent Office
Office européen des brevets

(11) EP 0 805 207 A1

(12) EUROPEAN PATENT APPLICATION

(43) Date of publication:
05.11.1997 Bulletin 1997/45

(51) Int. Cl.⁶: **C12N 15/62**, C07K 14/005,
C07K 14/195, C07K 14/37,
C07K 14/44, C07K 14/47,
C07K 14/52, A61K 39/295

(21) Application number: 96106935.8

(22) Date of filing: 02.05.1996

(84) Designated Contracting States:
AT BE CH DE DK ES FI FR GB GR IE IT LI LU MC
NL PT SE

(71) Applicant:
Gesellschaft für Biotechnologische
Forschung mbH (GBF)
D-38124 Braunschweig (DE)

(72) Inventors:
• Böhm, Waltraud
c/o Inst. für Med. Mikrobiologie
D-89069 Ulm (DE)

• Shirmbeck, Reinhold
c/o Inst.für Med.Mikrobiologie
D-89069 Ulm (DE)
• Reimann, Jörg
c/o Inst. für Med. Mikrobiologie der
D-89069 Ulm (DE)

(74) Representative:
Boeters, Hans Dietrich, Dr. et al
Patentanwälte Boeters & Bauer,
Bereiteranger 15
81541 München (DE)

(54) **Polycistronic expression plasmid for tumor rejection**

(57) The invention concerns a polycistronic expression plasmid encoding one or more viral antigens suitable for

(i) DNA based immunization of a cancer-bearing patient and/or (ii) transfection in vitro and/or in vivo of tumor cells of said patient.

Further, the invention concerns tumor cells of a cancer-bearing patient, wherein the tumor cells are transfected in vitro with a polycistronic expression plasmid according to the invention for injection into said patient.

In addition, the invention concerns a process for the production of a polycistronic expression plasmid according to the invention, wherein the following steps are provided:

(a) in vitro screening for a specific cytolytic T cell reactivity in peripheral blood of a cancer-bearing patient, wherein said reactivity is directed against one or more viral antigens,
(b) cloning one or more of said viral antigens and, optionally, one or more cytokines into a polycistronic expression vector.

Finally, the invention concerns a use of a polycistronic expression vector obtained according to the process according to the invention for an immunotherapy of cancer, wherein the following steps are provided:

(a) injecting in vivo the polycistronic expression vector into tumor cells of the cancer bearing patient; or

(b) isolating tumor cells from said patient, transfecting them in vitro with said polycistronic expression vector, and re-injecting the resulting genetically engineered tumor cells back into the patient; or
(c) transfecting in vitro tumor cells having been isolated from said cancer-bearing patient, with said polycistronic expression vector for re-injecting the resulting genetically engineered tumor cells back into the patient.

**Description**

INTRODUCTION

Active immunotherapy of malignant disease has been used in experimental models and in clinical oncology to prevent or suppress growth of primary tumors or of metastases. Tumor vaccines have been designed to preferentially stimulate major histocompatibility complex (MHC) class I-restricted cytotoxic T lymphocytes (CTL) because this T cell subset has been shown to reject syngeneic tumor grafts in many experimental systems (reviewed in 1-5). Stimulation of a CTL response requires the restricted presentation of epitopes from tumor-associated antigens (TAA) by tumor cells as well as the expression of an appropriate costimulator profile and selected cytokines. To enhance the immunogenicity of tumor cell vaccines, costimulator molecule-transfected tumor cells and paracrine cytokine adjuvants have been used (6-20).

Priming MHC class I-restricted CTL of defined epitope and restriction specificity to the hepatitis B surface antigen (HBsAg) in $H$-$2^d$ mice has been achieved using different protocols. CTL responses were induced by low doses of native, particulate HBsAg (21,22), by detergent-denatured, monomeric HBsAg (23), and by aggregated HBsAg (24) injected without adjuvants, and by the intramuscular injection of HBsAg-encoding expression plasmid DNA (25-27). These CTL specifically and efficiently lysed different, syngeneic tumor cell lines transfected with a HBsAg-encoding expression plasmid or pulsed with an antigenic peptide from HBsAg. A tumor cell line used in these experiments was the murine, methylcholanthrene-induced mastocytoma line P815 of DBA/2 ($H$-$2^d$) origin. Although immunogenic, cells of this line rapidly develop a tumor after transfer into immunocompetent, syngeneic hosts (28-35). The cloned, transfected subline P815/S expresses HBsAg and presents the $HBsAg_{28-39}$ epitope in the context of the MHC class I molecule $L^d$. P815/S transfectants secrete HBsAg, and non-transfected P815 cells efficiently process endocytosed, native HBsAg particles for class I-restricted presentation (36).

In syngeneic, naive or HBsAg-immune hosts the growth of non-transfected P815 tumors and P815/S tumors differed. In naive hosts, transfer of P815 cells induced rapidly growing tumors while the transfer of P815/S cells elicited tumors with a lower incidence. 'Spontaneous' rejection of P815/S tumors after transient growth was observed in some (> 5%) of the transplanted animals. In hosts primed to HBsAg by DNA immunization, growth of P815/S but not P815 tumors was efficiently suppressed. Non-immune hosts that had 'spontaneously' rejected transiently growing P815/S tumors, but not HBsAg-immune hosts that had rejected P815/S transplants (without evidence of transient tumor formation) were resistant to subsequent challenge by non-transfected P815 tumor cells. Hence, the former, but not under the latter conditions, apparently facilitated cross-priming of a TAA-specific, rejecting immune response.

According to one embodiment of the invention there is provided a polycistronic expression plasmid encoding one or more viral antigens suitable for

(i) DNA based immunization of a cancer-bearing patient and/or (ii) transfection in vitro and/or in vivo of tumor cells of said patient.

A polycistronic expression plasmid according to the invention encoding in addition one or more cytokines is preferred.

The polycistronic expression plasmid according to the invention may encode an antigen against which an anti-viral activity of the cancer-bearing patient is directed, and the plasmid might be provided for injection into tumor cells of said patient.

The polycistronic expression plasmid according to the invention may encode one or more antigens of common viral pathogens, especially measles, rubella, varicella, herpes virus and hepatitis B.

Another embodiment of the invention concerns tumor cells of a cancer-bearing patient, wherein the tumor cells are transfected in vitro with a polycistronic expression plasmid according to the invention for injection into said patient.

Another embodiment of the invention concerns a process for the production of a polycistronic expression plasmid according to the invention, wherein the process is characterized by the following steps:

(a) in vitro screening for a specific cytolytic T cell reactivity in peripheral blood of a cancer-bearing patient, wherein said reactivity is directed against one or more viral antigens,
(b) cloning one or more of said viral antigens and, optionally, one or more cytokines into a polycistronic expression vector.

Said process may be characterized in that the viral antigens are selected from the group of measles, rubella, varicella, herpes virus and hepatites B.

Still another embodiment of the invention concerns a use of a polycistronic expression vector obtained according to the process according to the invention for an immunotherapy of cancer, wherein the following steps are provided:

(a) injecting in vivo the polycistronic expression vector into tumor cells of the cancer bearing patient; or

(b) isolating tumor cells from said patient, transfecting them in vitro with said polycistronic expression vector, and re-injecting the resulting genetically engineered tumor cells back into the patient; or

(c) transfecting in vitro tumor cells having been isolated from said cancer-bearing patient, with said polycistronic expression vector for re-injecting the resulting genetically engineered tumor cells back into the patient.

Induction of a tumor-rejecting immune response is the aim in active immunotherapy of malignant disease. In a murine tumor (P815) model we demonstrate that targeting of an anti-viral T cell response to a growing tumor facilitates cross-priming of tumor-associated antigen (TAA)-specific, rejecting T cell-responses. Occasional 'spontaneous' rejections after transient subcutaneous growth of transfected P815/S tumors (expressing the hepatitis B surface antigen, HBsAg), but not of parental P815 tumors were observed. Growth of P815/S tumors (but not of P815 tumors) was efficiently suppressed by a $CD8^+$ cytotoxic T lymphocyte (CTL)-dependent immune mechanism in mice primed to HBsAg by DNA-immunization. In HBsAg-immune hosts, HBsAg-specific CTL were targeted to subcutaneously growing P815 tumors by intra-tumor injections of HBsAg-encoding plasmid DNA or viable P815/S cells; this treatment lead to tumor rejection in 70% to 80% of the tumor-bearing animals. P815-specific, $CD8^+$ CTL were detectable in all mice that rejected P815 tumors following this protocol, and all rejecting animals showed a $CD8^+$ CTL-dependent resistance to subsequent challenges by native, non-transfected P815 tumors. Targeting an established anti-viral ('strong') CTL response to a growing tumor hence facilitates cross-priming of a rejectinmg CTL response against ('weak') TAAs.

The inventors tested if P815 tumors are rejected when the potent HBsAg-specific CTL response primed by DNA immunization is targeted to the tumor. Targeting of CTL activity to the growing tumor was achieved by injecting into it, either HBsAg-encoding plasmid DNA, or HBsAg-expressing P815/S cells. Both treatment protocols resulted in suppression of the aggressive growth of P815 tumors in 70% to 80% of the treated, HBsAg-immune hosts. Focusing the anti-viral CTL response to a tumor aggressively growing in a syngeneic host thus facilitated tumor rejection in this model. Furthermore, P815-specific CTL reactivity and $CD8^+$ CTL-dependent resistance to challenges with naive P815 tumors was detected in all mice that rejected an established P815 tumor after this treatment protocol. These data thus indicate that focusing a potent anti-viral ('strong') CTL response to a growing tumor facilitates cross-priming of a rejecting T cell response to ('weak') TAA.

P815 cells form aggressively growing tumors in syngeneic DBA/2 hosts while cells of the transfected P815/S subline (that expressed HBsAg) display a low tumor-forming potential and a variable growth pattern. Naive animals rejected sometimes P815/S, but never P815 tumors following its transient growth. This suggested that HBsAg expression by P815 cells facilitated the generation of a rejecting immune response. Transfer of P815 cells transfected with vector DNA that encoded a functional neo gene or different fragments of the SV40 T-antigen established tumors with an incidence and growth patterns indistinguishable from that of parental P815 cells (table I; R.S., unpublished). Immunization of mice with HBsAg-encoding pRKS plasmid DNA (26) or other HBsAg-encoding vector constructs (25,27) primed class I-restricted CTL responses to HBsAg that eliminated transplanted P815/S cells. Hence, the CTL primed by DNA immunization not only lysed P815/S targets efficiently in vitro but also homed to growing P815/S tumors in vivo and eliminated most of them.

P815/S cell rejection was efficient in HBsAg-immune mice but not in non-immune mice because > 80% of HBsAg-immune mice rejected a graft of $10^4$ P815/S tumor cells without evidence of transient tumor formation after pRKS vector DNA immunization. In contrast, less than 30% of non-immune mice transplanted with $10^4$ P815/S cells rejected them. Compared to DNA immunization, priming of rejecting T cells by P815/S cell transfer was inefficient (data not shown).

The central issue of active immunotherapy of tumors is to prime rejecting, TAA-specific immunity. CTL were primed to TAA of P815 cells in a few non-immunized mice that rejected a transiently growing P815/S tumor; these animals resisted a subsequent challenge with $10^4$ non-transfected P815 cells (group 3 in table VI). Expression of 'strong' viral antigens or bacterial products by tumor cells facilitate priming of rejecting immune responses in some systems (9,42-45). In our system, we also found evidence for HBsAg-facilitated priming of a rejecting, TAA-specific immune response but this effect was inefficient. TAA-specific, protective immunity was not elicited during rejection of transplanted P815/S cells in pRKS DNA-immunized mice. In these immune mice, P815/S cells seem to be rapidly eliminated by HBsAg-specific CTL with little chance of cross-priming TAA-specific T cell reactivities. In contrast, CTL responses primed to β-galctosidase or ovalbumin by antigen-presenting dendritic cells have been reported to cross-prime TAA-specific, rejecting immune responses when restimulated by appropiately transduced tumor cells (46,47). This may be explained by the less efficient priming of CTL to the 'foreign' antigens in these systems. In the HBsAg system, the inefficient immune response to HBsAg (induced by P815/S transplantation) but not the efficient, rapidly rejecting CTL response (induced by DNA immunization) favored cross-priming of TAA-specific CTL.

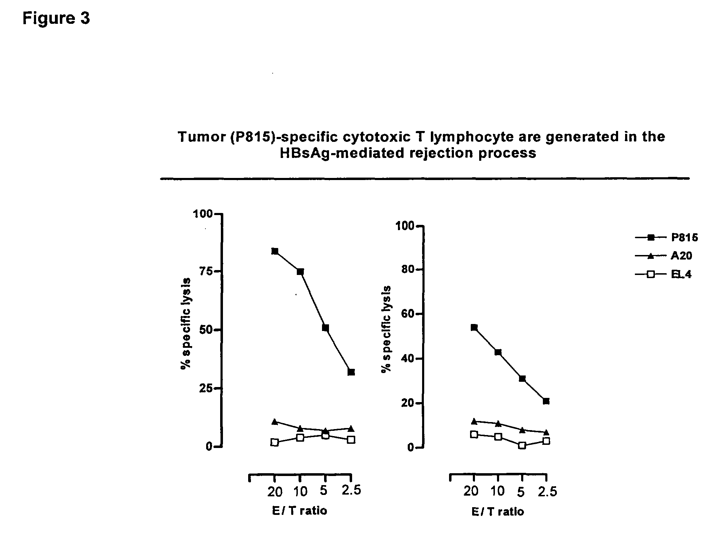

The CTL reactivity against HBsAg primed in mice by DNA immunization was focused to growing P815 tumors by intra-tumor injections of, either pRKS DNA, or viable P815/S transfectants. Intra-tumor injections of HBsAg particles did not facilitate tumor rejection (W.B. and R.S., unpublished data). P815 cells pulsed in vitro with HBsAg particles take up the exogenous antigen and process it for class I-restricted epitope presentation (36), this apparently does not take place in vivo. Successful were injections of pRKS vector DNA or P815/S transfectants into tumors growing in non-immune or immune mice. Injection of pRKS plasmid DNA into growing P815 tumors lead to rejection in 20-30% of non-immune mice. Similar data have been reported after injection of antigen-encoding expression plasmid DNA in other tumor models (48-50). In contrast, 70-80% of the P815 tumors growing in immune mice were rejected after pRKS plasmid DNA injection (table V and VI). All mice that had rejected tumors under this treatment protocol were resistant to a challenge transplant of P815 cells indicating cross-priming of rejecting, TAA-specific CTL responses (Fig. 3; table VII). Restimulation of an anti-HBsAg CTL response in the tumor apparently facilitated priming of a CTL response to TAA.

It is unknown how CTL are restimulated by viral antigen in tumors and how TAA-specific T cells are cross-primed. TAA-specific T cells may be primed by tumor cells coexpressing TAA and the viral antigen. Alternatively, they may be primed by professional APC (19,51) that have engulfed, either complete tumor cells, or antigenic vesicles (52) or apoptotic blebs (53) from tumor cells. Release of antigenic material by tumor cells may be facilitated by the attack of anti-viral CTL *in situ*. CTL restimulation within the tumor may enhance recruitement of APC into the tumor. Coss-priming may operate within the growing tumor, or it may take place in lymph nodes, a microenvironment that supports CTL priming to many cell types (54). HBsAg particles secreted by P815/S transfectants, P815 cells or antigenic material shed by these cells may be transported through lymphatics to the lymph nodes. The CTL response to HBsAg may provide essential cytokines or may induce costimulator molecules on the presenting tumor cells or activate APC that facilitate intermolecular 'spreading' of epitope recognition by CTL populations. Intra-and intermolecular 'spreading' of epitope recognition during ongoing T cell responses has been documented (55), and has also shown to develop during a CTL response against the thymoma EL4 (56). Interactions between HBsAg- and TAA-specific CD4$^+$ T cells and CD8$^+$ T cells, and APC are favorable conditions that support cross-priming of TAA-specific CTL.

Our data are relevant for the immunotherapie of cancer because they indicate a technique by which an anti-viral CTL response is used to facilitate cross-priming of rejecting TAA-specific CTL. HBsAg is an accepted vaccine in widespread use, and hence could be used for the immuntherapy of cancer in man. Alternatively, a naturally induced CTL reactivity against a common infectious agent diagnostically detected in the patient could be targeted to his/her tumor to facilitate cross-priming of rejecting immune responses. It will be of great interest to test if the encouraging results in animal models can be reproduced in cancer patients.

It follows a detailed description of the invention based on figures, materials and methods and experimental results.

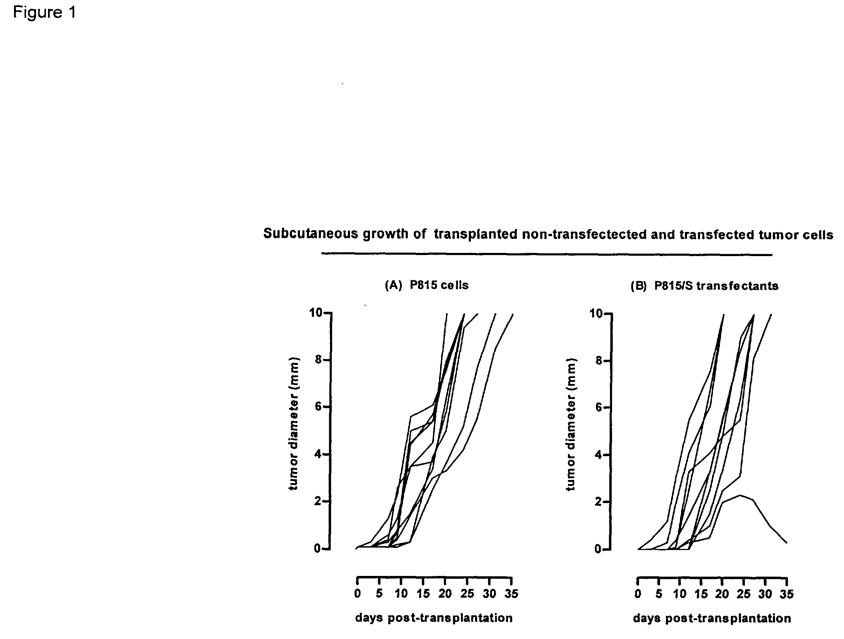

**Figure 1.** *Subcutaneous growth of non-transfected P815 or transfected P815/S cells in syngeneic DBA/2 hiosts.* Mice were subcutaneously injected with $10^3$ or $10^4$ non-transfected P815 cells(A) or transfected P815/S cells (B). Tumor growth was measured every second or third day. For 10 mice developing tumors, the mm tumor diameter (y-axis) at different time points (days) after tumor cell transplantation (x axis) is plotted.

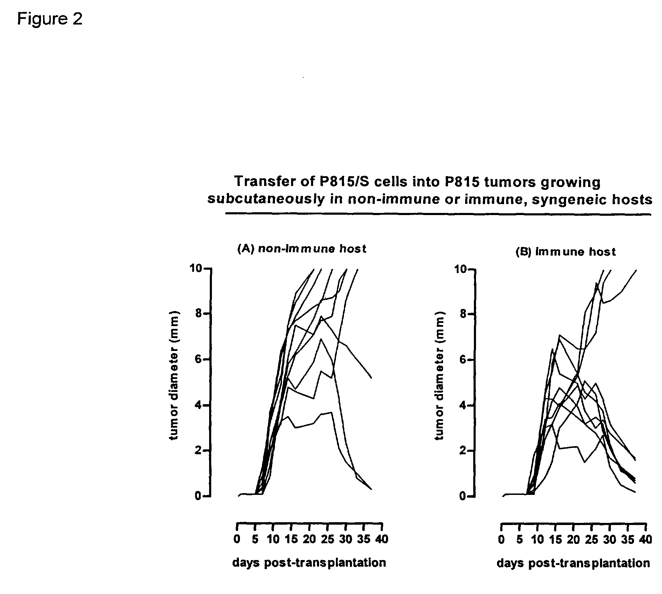

**Figure 2.** *Repeated injections of HBsAg-encoding pRKS vector DNA into growing P815 tumors leads to tumor regression* . P815 cells ($10^4$ per mouse) were subcutaneously transplanted into mice which were injected intramuscularly 3 weeks previously with 100 µg pRK vector DNA without insert (A) or HBsAg-encoding pRKS vector DNA (B). Mice were treated by repeated injections of HBsAg -encoding pRKS plasmid DNA into the tumor. The mm tumor diameter (y-axis) at different time points (days) post tumor cell inoculation (x axis) is plotted for 10 individual mice from a representative experiment. In (A) 3/10 tumor-bearing mice rejected the tumor; in (B), 7/10 tumor-bearing mice rejected the tumor.

**Figure 3.** *P815-specific CD8$^+$ cytotoxic T lymphocytes are generated during the HBsAg-facilitated rejection of P815 tumors*. Mice with P815 tumors that were primed to HBsAg by pRKS vector DNA immunization were treated by intra-tumour injections of pRKS plasmid DNA. Spleen cells obtained from mice that rejected the tumor were cocultured for 5 days with irradiated P815 cells. The specific cytolytic reactivity of effector cells harvested from these cultures was tested against P815 targets (˩), A20 targets ( ), and EL4 targets (σ) in a 4h $^{51}$Cr release assay. Cytolytic effector cells were eliminated by treatment with anti-CD8 mAb and complement. Data from 2 representative mice are shown.

*Abbreviations*: CTL, cytotoxic T lymphocyte; APC, antigen-presenting cell; MHC, major histocompatibility complex; HBV, hepatitis B virus; HBsAg, hepatitis B surface antigen; HBsAg-particle, 22 nm spheres composed of about 100 HBsAg proteins; TAA, tumor-associated antigen.

MATERIALS AND METHODS

*Mice*. DBA/2JBom mice (H-2$^d$) were bred and kept under specific-pathogen-free conditions in the animal colony of Ulm University (Ulm, FRG). Breeding pairs of these mice were obtained from Bomholtgard (Ry, DK). Male and female mice were used at 12-16 wks of age.

*HBsAg-encoding expression vector pRKS used for DNA immunization*. The construction of the expression plasmid pRKS containing the small HBsAg, subtype ayw, has been described (26). The HBsAg-encoding gene is expressed under CMV promoter control in this construct. Plasmid DNA used for immunization was purified by anion exchange chromatography using the Qiagen maxi prep kit (Qiagen, Hilden, FRG). 'Naked' plasmid DNA suspended at 1 µg/µl in PBS was injected. The plasmid pRKS contains the HBsAg gene; the vector pRK has no insert. Similar results were obtained with different HBsAg-encoding expression plasmid constructs (27).

*Cell lines*. The H-2$^d$ mastocytoma cell line P815 (TIB64) was obtained from the American Tissue Culture Collection (ATCC, Rockville, MD). The bovine papilloma virus-based vector BMGNeo (37), a generous gift from Drs. Y. Karasuyama and F. Melchers (Basel, Switzerland) was used to construct the BMG/HBS expression plasmid (22). BMG/HBS vector DNA, or non-recombinant BMGNeo vector DNA were transfected into P815 cells as described (21,22). Stable expression of HBsAg was demonstrated in cloned and subcloned P815/S cells (22). Neo-resistent P815/BMG cells were transfected with BMGNeo vector DNA without insert. The B lymphoma cell line A20 (TIB-208) of BALB/c origin and the EL4 thymoma cell line (TIB-39) of C57BL/6 (H-2$^b$) origin was obtained from the ATCC. Hybridomas producing the anti-CD4 mAb YTS 191.1 or the anti-CD8 mAb YTS 169.4 were a generous gift from Dr.S.H.Kaufmann (Ulm, FRG).

*Nucleic acid immunization*. We injected 50 ◊ l PBS containing 50 ◊ g plasmid DNA into each regenerating tibialis anterior muscle 5 days after the injection of cardiotoxin (Latoxan, Rosans, France) as previously described (38,39). All mice received bilateral intramuscular injections once. Mice injected with pRKS plasmid DNA are designated 'immune' to HBsAg. These animals developed a potent HBsAg-specific CTL response and high serum antibody levels to HBsAg (26,27). Non-injected mice, or mice injected with pRK plasmid DNA without insert are designated 'non-immune'.

*Subcutaneous transplantation of tumor cells into syngeneic DBA/2 hosts*. P815, P815/BMG and P815/S cells were adapted to subcutaneous growth in DBA/2 mice. Cells were harvested from tumors. Single cell suspensions were prepared in serum-free PBS. Titrated numbers of cells were injected in 200 μl PBS subcutaneously into the left lateral flank of age- and sex-matched mice (5 to 8 animals per group). Tumor growth was measured every second or third day. Mice bearing tumors with a diameter greater than 1 cm were sacrificed.

*Intratumor injections*. Into tumors with a diameter of about 0.4 cm, we injected, either 50 μl PBS containing 20 μg DOTAP-incorporated plasmid DNA, or 50 μl PBS containing 10$^4$ viable P815/S cells. In some experimental series, these injections were repeated three times every second day. Tumor growth was followed until, either the tumor diameter reached 1 cm, or the tumor had regressed to a macroscopically undetectable size.

*In vivo suppression of CD4$^+$ or CD8$^+$ T cells in mice*. CD4$^+$ or CD8$^+$ T cell subsets were suppressed in mice by repeated injections of the anti-CD4 mAb YTS 191.1 or the anti-CD8 mAb YTS 169.4 (40). At day 3 and day 1 before, and day 1 after, tumor cell transplantation, mice were intraperitoneally injected with 200 μl PBS containing 200 μg purified antibody. Flow cytometric analyses of splenic and/or peripheral blood mononuclear cell populations demonstrated that 87% to 98% of T cells expressing the respective phenotype were deleted in treated mice.

*Cytotoxic assay*. Spleen cells or lymph node cells from mice were suspended in ∟-MEM tissue culture medium supplemented with 10 mM Hepes buffer, 5 x 10$^{-5}$ M 2-ME, antibiotics and 10% v/v FCS (Pan Systems, Aidenbach, FRG). We cocultured 3 x 10$^7$ responder cells with 1.5 x 10$^6$ P815 cells (irradiated with 20.000 rad) in 10 ml medium in upright 25 cm$^2$ tissue culture flasks in a humidified atmosphere/7% CO$_2$ at 37¡C. Cytotoxic effector populations were harvested after 5 days of *in vitro* culture and washed twice. Serial dilutions of effector cells were cultured with 2 x 10$^3$ $^{51}$Cr-labeled targets in 200 μl round-bottom wells. Specific cytolytic activity of cells was tested in short-term $^{51}$Cr-release assays against different targets. After a 4 h incubation at 37¡C, 100 μl of supernatant were collected for ||-radiation counting. The percentage specific release was calculated as [(experimental release - spontaneous release)/(total release - spontaneous release)] x 100. Total counts were measured by resuspending target cells. Spontaneously released counts were always less than 15 % of the total counts. Data shown are the mean of triplicate cultures. The SD of triplicate data was always less than 15% of the mean.

RESULTS

*Subcutaneous growth of non-transfected P815 and transfected P815/S cells in syngeneic hosts*. P815 mastocytoma cells adapted to growth *in vivo* were subcutaneously injected into DBA/2 mice. From a small inoculum of 10$^2$ cells, a rapidly growing, subcutaneous tumor developed in 90% of the transplanted animals (table I, group 1). All mice transplanted with 10$^4$ P815 cells developed tumors within 2 weeks post-transplantation (table I, group 3). The tumors reached a diameter of 1 cm in the third or fourth week post-transplantation in mice injected with 10$^2$, 10$^3$ or 10$^4$ P815 cells (fig. 1A). We further transplanted the cloned, transfected P815/S subline that expresses HBsAg (22). P815/S cells efficiently present the HBsAg epitope to class I-restricted CTL in vitro and secrete HBsAg particles (detectable in ELISA) into the medium that are efficiently endocytosed by P815 cells and processed for class I-restricted epitope presentation in an alternative pathway (36). After subcutaneous injection of 10$^2$, 10$^3$ or 10$^4$ P815/S cells, only a fraction of 25% to 75% of the transplanted mice developed a tumor (table I, group 7-9). Transient tumor growth followed by subsequent rejection of tumors was observed in some mice (figure 1B). Mice showing regressing P815/S tumors were found in groups transplanted with all three doses of tumor cells but represented less than 5% of all transplanted mice. P815/S cells forming tumors in DBA/2 mice expressed HBsAg as this viral antigen was easily detectable in immunoprecipitates of cells harvested from growing tumors (data not shown). Hence, growth of these transfectants in the syngeneic, immunocompetent host did not select for escape variants that suppressed expression of the viral antigen. Mice in a control group were transplanted with P815/BMG cells transfected with vector DNA without insert. Subcutaneous growth of P815/BMG cells in DBA/2 mice was indistinguishable from that of non-transfected P815 cells (compare groups 1-3 and 4-6 in table I).

*HBsAg-specific CTL primed by nucleic acid immunization reject P815/S tumor grafts.* Intramuscular injection of HBsAg-encoding pRKS plasmid DNA primes a specific antibody response and a potent response of class I-restricted CTL (25,26). Non-transfected P815 or transfected P815/S cells were injected subcutaneously into DBA/2 mice immunized, either with pRK vector DNA not containing an insert, or with pRKS vector DNA containing the HBsAg-encoding gene. Rapid growth of non-transfected P815 cells was observed in all DNA-inoculated mice (table II, group 1-6). Growth of transfected P815/S cells in mice injected with pRK vector DNA without insert was indistinguishable from P815/S tumor cell growth in non-injected mice (compare groups 7-9 in table I and II). In contrast, no growth of P815/S tumors was observed in most mice immune to HBsAg after inoculation with pRKS vector DNA (table II, groups 10-12). DNA vaccination thus stimulated an immune response that rejected even a high inoculum of $10^4$ P815/S tumor cells in most syngeneic hosts.

Class I-restricted CD8+ CTL mediate rejection of P815/S cells in HBsAg-immune mice. The CD4+ or the CD8+ T cell subset were deleted in vivo by repeated treatment with monoclonal antibodies prior to and after P815/S tumor cell transplantation. Depletion of CD8+ T cells (table III, group 3) impaired the ability of immune mice to reject P815/S cells while the depletion of CD4+ T cells had only a marginal effect (table III, group 2). Hence, CD8+ T cells primed by DNA vaccination to HBsAg are involved in the rejection of syngeneic P815/S cells.

*Rejection of P815/S tumor cells in non-immune, but not in HBsAg-immune mice cross-primes a TAA-specific immune response that protects the host against a P815 tumor cell graft.* P815 cells express CTL-defined TAA (28,31-35,41). We asked if the T cell responses rejecting P815/S tumors in non-immune or immune hosts display exclusive specificity for HBsAg, or, if P815-specific CTL are cross-primed during the rejection process that contribute to the P815/S tumor rejection. We selected 6 mice for these experiments that were primed to HBsAg by DNA-based vaccination and had rejected an inoculum of $10^4$ P815/S cells. Four weeks post-transplantation, these animals were challenged by a subcutaneous injection of $10^4$ non-transfected P815 cells. All 6 mice developed a tumor (table IV, group 2). The growth of P815 tumors in non-immune control mice (table IV, group 1), and in immunized, P815/S tumor-rejecting mice (table IV, group 2) was similar. Hence, the rejecting response against P815/S cells in HBsAg-immune hosts is directed against the viral antigen (HBsAg) but not against P815-specific TAA. If P815 TAA-specific immune reactivities are generated during the rejection of P815/S transplants in immune mice, they do not protect the host against the aggressive growth of a subsequent P815 tumor graft.

Some non-immune DBA/2 mice transplanted with P815/S cells 'spontaneously' rejected the transplant after transient tumor growth (fig. 1B). Four mice that had rejected a graft of $10^4$ P815/S cells after transient growth of a tumor were challenged by a subcutaneous injection of $10^4$ non-transfected P815 cells. All four mice rejected the P815 cell transplant that lead to growth of an aggressive tumor in all non-pretreated animals (table IV, groups 1 and 3). The immune response to HBsAg primed by P815/S rejection seems to have facilitated cross-priming of a rejecting, P815-specific immune response.

*Injections of HBsAg-encoding plasmid DNA into growing P815 tumors target the HBsAg-specific immune reactivity of primed mice to the tumor and facilitates its rejection.* We transplanted P815 tumor cells into non-immune mice (not pretreated, or pretreated by an intramuscular inoculation of pRK vector DNA without insert). Into subcutaneously growing tumors, we injected liposome-incorporated, HBsAg-encoding pRKS plasmid DNA to prime an anti-HBsAg-specific immune response within the tumor. Data from representative experiments are shown in figure 2A and table V. DNA injections into tumors growing in non-immune mice lead to tumor regression in 36 % (10/28) of the treated mice (table V, group 2) indicating that an immune response primed in a growing tumor can suppress its growth in a fraction of the animals.

In the next series of experiments, we vaccinated mice against HBsAg by an intramuscular inoculation of pRKS vector DNA three weeks prior to tumor cell transplantation. These animals were transplanted with $10^4$ P815 cells and a tumor developed in 29 out of 35 mice (table V, group 3). Into P815 tumors growing in these 29 mice, we injected pRKS vector DNA. In 19 out of 29 (66%) tumor-bearing, HBsAg-immune mice, tumor regression was apparent in the course of this treatment. Regression and rejection were evident by a decrease in tumor diameter, the appearance of a central necrosis, and signs of inflammation. No evidence of tumor growth was evident during an observation period of three months after the rejection. Hence, an anti-viral immune response (induced by DNA immunization) focused to a growing tumor by transient *in vivo* transfection (following intratumor injections of liposome-incorporated pRKS vector DNA) can initiate tumor rejection.

*Transfer of P815/S cells into P815 tumors growing in HBsAg-immune, syngeneic hosts facilitates tumor rejection.* P815 tumor cells transiently transfected *in vivo* with HBsAg-encoding vector DNA can apparently facilitate tumor rejection. These data were supported, when we injected P815/S cells into P815 tumors growing in non-immune or HBsAg-immune hosts. The repeated transfer of $10^4$ viable P815/S cells into P815 tumors subcutaneously growing in non-immune hosts lead to tumor regression in 20% of the animals. This treatment was more efficient in mice vaccinated against HBsAg by intramuscular pRKS vector DNA inoculations prior to the tumor cell transfers. When 13 immune animals bearing growing P815 tumors were treated by intra-tumor injections of P815/S cells, tumor regression was apparent in 10 (77%) of the mice (table VI, group 3). These data were similar to those found with the intra-tumor DNA treatment protocol. The findings support the idea that an anti-viral immune response recruited into a growing tumor by intra-tumor antigen expression can initiate a tumor-rejecting immune response in a majority of treated mice. Further experiments were designed to address the questions: (i) is the rejecting immune response HBsAg-or/and P815-specific; and (ii) what is the rejecting immune mechanism.

*The rejecting immune response is P815-specific*. All mice that had rejected P815 tumors following intratumor injections of pRKS vector DNA or P815/S cells were challenged 5 weeks post-rejection by the subcutaneous injection of $10^4$ P815 tumor cells. In none of the rejecting mice, a tumor developed (table V and VI). In contrast, 80-100% of naive mice transplanted with the same number of P815 cells developed a tumor (table V and VI). Hence, a P815-specific, rejecting immune response is cross-primed during the anti-HBsAg response in the tumor.

*P815 tumor rejection is mediated by CD8$^+$ CTL*. In mice that had rejected a P815 mastocytoma we detected *in vitro* CD8$^+$ T cells with specific cytolytic reactivity against P815 targets (Fig. 3). These CTL did not lyse other syngeneic or allogeneic targets, *i.e.*, H-2$^d$ A20 B lymphoma cells or H-2$^b$ EL4 thymoma cells. CD8$^+$ T cells mediate P815 cell rejection. CD8$^+$ or CD4$^+$ T cells were depleted *in vivo* in DNA-immunized mice that had rejected a growing P815 tumor after intra-tumor injections of HBsAg-encoding DNA or of P815/S cells. When these mice were challenged by a subcutaneous injection of $10^4$ P815 cells, a tumor developed in 4 out of 5 CD8$^+$ T cell-depleted animals, but only in 1 out 5 CD4$^+$ T cell-depleted mice (table VII). CD8$^+$ T cell depletion thus allowed growth of P815 tumor cells in immune mice. P815-specific CD8$^+$ CTL cross-primed during the immune response to HBsAg within the tumor therefore play a role in rejecting this aggressive tumor.

Table I

| group | cell line[a] | # cells transferred[b] | tumor incidence[c] | |
|---|---|---|---|---|
| Subcutaneous growth of transfected and non-transfected P815 cells in a syngeneic host | | | | |
| | | | % | tumor- bearing/trans- planted mice |
| 1 | P815 | $10^2$ | 90 | 31/34 |
| 2 | P815 | $10^3$ | 91 | 28/31 |
| 3 | P815 | $10^4$ | 100 | 16/16 |
| 4 | P815/BMG | $10^2$ | 83 | 10/12 |
| 5 | P815/BMG | $10^3$ | 83 | 10/12 |
| 6 | P815/BMG | $10^4$ | 100 | 12/12 |
| 7 | P815/S | $10^2$ | 25 | 6/24 |
| 8 | P815/S | $10^3$ | 44 | 14/32 |
| 9 | P815/S | $10^4$ | 75 | 18/24 |

[a] non-transfected P815 cells (P815), P815 cells transfected with with BMGneo vector without insert (P815/BMG), or P815 cells transfected with the HBsAg-encoding BMG/HBS vector (P815/S) were transplanted into DBA/2 mice
[b] $10^2$, $10^3$, or $10^4$ cells suspended in 0.2 ml PBS were injected subcutaneously into the left lateral flank
[c] tumor growth was monitored every second or third day. The % of mice developing a tumor, and the number of tumor-bearing mice /total number of transplanted mice are listed. Pooled data from 4 independent experiments are shown

Table II

| group | cells transferred[a] | | HBsAg pre-immune[b] | tumor incidence[c] | |
|---|---|---|---|---|---|
| | cell line | # | | % | tumor- bearing/trans- planted mice |
| 1 | P815 | $10^2$ | - | 83 | 10/12 |
| 2 | P815 | $10^3$ | - | 91 | 11/12 |
| 3 | P815 | $10^4$ | - | 100 | 12/12 |
| 4 | P815 | $10^2$ | + | 86 | 12/14 |
| 5 | P815 | $10^3$ | + | 91 | 13/14 |
| 6 | P815 | $10^4$ | + | 100 | 14/14 |
| 7 | P815/S | $10^2$ | - | 38 | 6/16 |
| 8 | P815/S | $10^3$ | - | 38 | 6/16 |
| 9 | P815/S | $10^4$ | - | 63 | 10/16 |
| 10 | P815/S | $10^2$ | + | 0 | 0/16 |
| 11 | P815/S | $10^3$ | + | 13 | 2/16 |
| 12 | P815/S | $10^4$ | + | 13 | 2/16 |

**Anti-HBsAg immune reactivity primed by nucleic acid immunization rejects P815/S tumor grafts**

[a] non-transfected P815 cells (P815) or P815 cells transfected with the HBsAg-encoding vector BMG/HBS (P815/S) were transplanted into DBA/2 mice; #: $10^2$, $10^3$, or $10^4$ cells suspended in 0.2 ml PBS were injected subcutaneously into the left lateral flank
[b] all DBA/2 mice were intramuscularly injected with 100 μg plasmid DNA three weeks prior to the subcutaneous tumor cell transplantation. Mice were injected with, either pRK vector DNA without insert (-), or pRKS vector DNA containing the HBsAg-encoding gene (+)
[c] tumor growth was monitored every second or third day. The % of mice developing a tumor, and the number of tumor-bearing mice /total number of transplanted mice are listed

Table III

**CD8[+] T cells mediate the rejection of P815/S tumor cells transplanted into HBsAg-immune, syngeneic hosts**

| group | mice treated with[1] | tumor incidence[2] | |
|---|---|---|---|
| | | % | incidence |
| 1 | - | 13 | 1/8 |
| 2 | anti-CD4 mAb YTS 191.1 | 38 | 3/8 |
| 3 | anti-CD8 mAb YTS 169.4 | 88 | 7/8 |

[1] all DBA/2 mice were immunized with pRKS plasmid DNA three weeks prior to antibody treatment and tumor cell transplantation. Mice in group 2 and 3 were treated by three intraperitoneal injections of 200 μg anti-CD4 mAb YTS 191.1 or anti-CD8 mAb YTS 169.4 at day 3 and day 1 before, and day 1 after subcutaneous injection of $10^4$ P815/S cells
[2] tumor growth was monitored for 6 weeks post-transplantation

Table IV

| Recruitment of a tumor (P815)-specific, rejecting immune response during the rejection of P815/S tumors in non-immune or HbsAg-immune mice | | | | | |
|---|---|---|---|---|---|
| host[a] | | | tumor challenge[b] | tumor incidence[c] | |
| group | DNA i.m. | rejected tumor | | % | tumor-bearing/transplanted mice |
| 1 | pRK | - | P815 $10^4$ s.c. | 100 | 6/6 |
| 2 | pRKS | P815/S $10^4$ s.c. | P815 $10^4$ s.c. | 100 | 6/6 |
| 3 | - | P815/S $10^4$ s.c. | P815 $10^4$ s.c. | 0 | 0/4 |

[a] DBA/2 mice were injected intramuscularly (i.m.) with 100 µg pRK vector DNA (group 1, -) or 100 µg pRKS vector DNA (group 2, +). Three weeks post-immunzation, mice in group 2 were transplanted subcutaneously with $10^4$ P815/S cells; no growth of P815/S tumors was detectable in mice in group 2 which were challenged with P815 cells. Mice in group 3 were not injected with DNA, transplanted with $10^4$ P815/S tumor cells, and had rejected the tumor after a transient growth to a size of 3 to 7 mm in diameter
[b] four weeks after the rejection of P815/S cells (in group 2 and 3), $10^4$ non-transfected P815 cells were subcutaneously injected into the mice
[c] appearance and growth of P815 tumors was monitored every second or third day. The number of tumor-bearing mice / total number of transplanted mice (and the %) are listed

Table V

| Repeated injections of HBsAg-encoding pRKS vector DNA into P815 tumors growing in DNA immunized mice induce a rejecting anti-P815 tumor immunity | | | | | | |
|---|---|---|---|---|---|---|
| host[a] | | | | primary tumor[e] | | P815 tumor challenge[f] |
| group | HBsAg immune[b] | P815 s.c.[c] | pRKS i.t.[d] | tumor-bearing / transplanted | tumor-regress-ing / tumor-bear-ing | tumor-rejecting |
| 1 | - | + | - | 26/28 (93) | 0/26 (0) | ND |
| 2 | - | + | + | 28/35 (80) | 10/28 (36) | 10/10 |
| 3 | + | + | + | 29/35 (83) | 19/29 (66) | 19/19 |
| ND, not done | | | | | | |

[a] Pooled data from two independent experiments are shown
[b] mice were injected i.m. with 100 µg plasmid DNA of the vector pRK (-) or pRKS (+) three weeks prior to tumor cell transplantation
[c] $10^3$ or $10^4$ P815 cells were subcutaneously transferred into mice
[d] DOTAP-incorporated pRK (-) or pRKS (+) plasmid DNA (20 µg) was injected into growing tumors (i.t.) every second/third day
[e] growth of P815 tumor was monitored for 8 weeks. The number of tumor-bearing mice in the group of transplanted mice (%) is shown. The number of regressing tumors among the tumor-bearing mice (%) is listed
[f] mice that had completely rejected the tumor were challenged with a subcutaneous transplantation of $10^4$ P815 cells 8 weeks after the rejection

Table VI

| host[a] | | | | primary tumor[e] | | P815 tumor challenge[f] |
|---|---|---|---|---|---|---|
| group | HBsAg immune[b] | P815 s.c.[c] | P815/S i.t. | tumor-bearing / transplanted | tumor-regress-ing / tumor-bear-ing | tumor-rejecting |
| 1 | - | + | - | 13/16 (81) | 0/13 (0) | ND |
| 2 | - | + | + | 15/16 (94) | 3/15 (20) | 3/3 |
| 3 | + | + | + | 13/16 (81) | 10/13 (77) | 10/10 |
| ND, not done | | | | | | |

Repeated injections of P815/S cells into P815 tumors growing in syngeneic hosts induce a rejecting anti-P815 tumor immunity

[a] DBA/2 mice

[b] three weeks prior to tumor cell transplantation, mice were injected i.m. with 100 µg plasmid DNA of the vector pRK without insert (-), or the vector pRKS encoding HBsAg (+)

[c] $10^3$ or $10^4$ P815 cells were subcutaneously transferred to mice

[d] viable P815/S cells ($10^4$ cells in 50 µl PBS) were injected into growing tumors (i.t.) every second/third day

[e] growth of P815 tumor was monitored for 8 weeks. The number of tumor-bearing mice in the group of transplanted mice (%) is shown. The number of regressing tumors among the tumor-bearing mice (%) is listed

[f] mice that had completely rejected the tumor were challenged with a subcutaneous transplantation of $10^4$ P815 cells 8 weeks after the rejection

Table VII

| group | treatment of rejecting mice[a] | tumor incidence[b] |
|---|---|---|
| 1 | - | 0/5 |
| 2 | anti-CD4 mAb YTS 191.1 | 1/5 |
| 3 | anti-CD8 mAb YTS 169.4 | 4/5 |

P815-specific CD8[+] T cells recruited into the anti-HBsAg immune response mediate tumor rejection

[a] DBA/2 mice were injected intramuscularly (i.m.) with 100 µg pRKS vector DNA. Three weeks post-immunzation mice were transplanted subcutaneously with $10^4$ P815 cells. Into growing tumors, DOTAP-incorporated pRKS plasmid DNA (20 µg) was injected every second/third day. Growth of P815 tumors was monitored every second day for 8 weeks. Mice that completely rejected the tumor were selected for this experiment. They were challenged by a subcutaneous injection of $10^4$ P815 cells 8 weeks after the rejection of the P815 tumor. Mice in group 2 and 3 were treated by three intraperitoneal injections of 200 µg anti-CD4 mAb YTS 191.1 or anti-CD8 mAb YTS 169.4 at day 3 and day 1 before, and day 1 after the P815 cell transfer

[b] tumor growth was monitored for 6 weeks post-transplantation. The number of tumor-bearing / transplanted mice is shown

Table VIII

| group | tumor cell transplantation[a] | | | rejection[b] | | |
|---|---|---|---|---|---|---|
| | pre- immunization: pRKS plasmid DNA i.m. | transplanted tumor cells | pRKS plasmid DNA: intra-tumor injections | efficiency | specificity | |
| | | | | | HBsAg | P815-TAA |
| 1 | - | P815/S | - | (+) | + | + |
| 2 | + | P815/S | - | +++++ | + | - |
| 3 | - | P815 | - | - | - | - |
| 4 | + | P815 | - | - | - | - |
| 5 | - | P815 | + | ++ | + | + |
| 6 | + | P815 | + | +++ | + | + |

HBsAg-facilitated rejection of P815 tumors

[a] *protoool*: DBA/2 mice were pre-immunized by an intramuscular injection of pRKS plasmid to HBsAg (+), or injected i.m. with the control plasmid pRK (-). Three weeks after the DNA injection, P815 or P815/S tumor cells were transplanted subcutaneously. pRKS plasmid DNA (+), or pRK plasmid DNA (-) was injected repeatedly into the growing tumors
[b] failure of engraftment, or rejection of tumor cells was monitored for 8 weeks. The relative efficiency of rejection, and the (viral HBsAg-specific versus tumor-specific) specificity of the rejecting CTL response are listed

REFERENCES

1. **Boon, T.** 1992. Toward a genetic analysis of tumor rejection antigens. *Adv. Cancer Res.* 58:177.

2. **Urban, J. L. and H. Schreiber**. 1992. Tumor antigens. *Annu. Rev. Immunol. 10*:617.

3. **Boon, T., J. C. Cerottini, B. Van den Eynde, P. Van der Bruggen, and A. van Pel**. 1994. Tumor antigens recognized by T lymphocytes. *Annu. Rev. Immunol. 12*:337.

4. **van Pel, A., P. Van der Bruggen, P. G. Coulie, V. G. Brichard, B. Lethe, B. Van den Eynde, C. Uyttenhove, J. C. Renauld, and T. Boon**. 1995. Genes coding for tumor antigens recognized by cytolytic T lymphocytes. *Immunol. Rev. 145*:229.

5. **Boon, T. and P. Van der Bruggen**. 1996. Human tumor antigens recognized by T lymphocytes. *J. Exp. Med. 183*:725.

6. **Asher, A. L., J. J. Mule, A. Kasid, N. P. Restifo, J. C. Salo, C. M. Reichert, G. Jaffe, B. Fendly, M. Kriegler, and S. A. Rosenberg**. 1991. Murine tumor cells transduced with the gene for tumor necrosis factor-alpha. Evidence for paracrine immune effects of tumor necrosis factor against tumors. *J. Immunol. 146*:3227.

7. **Barth, R. J., Jr., J. J. Mule, P. J. Spiess, and S. A. Rosenberg**. 1991. Interferon gamma and tumor necrosis factor have a role in tumor regressions mediated by murine CD8+ tumor-infiltrating lymphocytes. *J. Exp. Med. 173*:647.

8. **Jicha, D. L., J. J. Mule, and S. A. Rosenberg**. 1991. Interleukin 7 generates antitumor cytotoxic T lymphocytes against murine sarcomas with efficacy in cellular adoptive immunotherapy. *J. Exp. Med. 174*:1511.

9. **Chen, L., S. Ashe, W. A. Brady, I. Hellström, K. E. Hellström, J. A. Ledbetter, P. McGowan, and P. S. Linsley**. 1992. Costimulation of antitumor immunity by the B7 counterreceptor for the T lymphocyte molecules CD28 and CTLA-4. *Cell 71*:1093.

10. **Chen, L., P. S. Linsley, and K. E. Hellström**. 1993. Costimulation of T cells for tumor immunity. *Immunol. Today 14*:483.

11. **Townsend, S. E. and J. P. Allison**. 1993. Tumor rejection after direct costimulation of CD8+ T cells by B7-transfected melanoma cells. *Science 259*:368.

12. **Chen, L., P. McGowan, S. Ashe, J. Johnston, Y. Li, I. Hellström, and K. E. Hellström**. 1994. Tumor immunogenicity determines the effect of B7 costimulation on T cell-mediated tumor immunity. *J. Exp. Med. 179*:523.

13. **Tepper, R. I. and J. J. Mule**. 1994. Experimental and clinical studies of cytokine gene-modified tumor cells. *Hum. Gene Ther. 5*:153.

14. **Gajewski, T. F., J. C. Renauld, A. van Pel, and T. Boon**. 1995. Costimulation with B7-1, IL-6, and IL-12 is sufficient for primary generation of murine antitumor cytolytic T lymphocytes in vitro. *J. Immunol. 154*:5637.

15. **Pardoll, D. M**. 1995. Paracrine cytokine adjuvants in cancer immunotherapy. *Annu. Rev. Immunol. 13*:399.

16. **Sule Suso, J., F. Arienti, C. Melani, M. P. Colombo, and G. Parmiani**. 1995. A B7-1-transfected human melanoma line stimulates proliferation and cytotoxicity of autologous and allogeneic lymphocytes. *Eur. J. Immunol. 25*:2737.

17. **Wu, T. C., A. Y. Huang, E. M. Jaffee, H. I. Levitsky, and D. M. Pardoll**. 1995. A reassessment of the role of B7-1 expression in tumor rejection. *J. Exp. Med. 182*:1415.

18. **Hellström, K. E., I. Hellström, and L. Chen**. 1996. Can costimulated tumor immunity be therapeutically efficacious? *Immunol. Rev 145*:123.

19. **Huang, A. Y., A. T. Bruce, D. M. Pardoll, and H. I. Levitsky**. 1996. Does B7-1 expression confer antigen-presenting cell capacity to tumors *in vivo* ? *J. Exp. Med. 183*:769.

20. **Li, Y., K. E. Hellström, S. A. Newby, and L. Chen**. 1996. Costimulation by CD48 and B7-1 induces immunity against poorly immunogenic tumors. *J. Exp. Med. 183*:639.

21. **Schirmbeck, R., K. Melber, T. Mertens, and J. Reimann**. 1994. Antibody and cytotoxic T-cell responses to soluble hepatitis B virus (HBV) S antigen in mice: implication for the pathogenesis of HBV-induced hepatitis. *J. Virol. 68*:1418.

22. **Schirmbeck, R., K. Melber, A. Kuhröber, Z. A. Janowicz, and J. Reimann**. 1994. Immunization with soluble hepatitis B virus surface (S) protein particles elicits murine H-2 class I-restricted CD8+ cytotoxic T lymphocyte responses in vivo. *J. Immunol. 152*:1110.

23. **Schirmbeck, R., K. Melber, T. Mertens, and J. Reimann**. 1994. Selective stimulation of murine cytotoxic T cell and antibody responses by particulate or monomeric hepatitis B virus surface (S) antigen. *Eur. J. Immunol. 24*:1088.

24. **Schirmbeck, R., W. Böhm, K. Melber, and J. Reimann**. 1995. The *in vivo* immunogenicity and the processing pattern of 'exogenous' aggregated or particulate hepatitis B surface antigen for class I-restricted T cells are strikingly different. *J. Immunol. 155*:4676.

25. **Davis, H. L., R. Schirmbeck, J. Reimann, and R. G. Whalen**. 1995. DNA-mediated immunization in mice induces a potent MHC class I-restricted cytotoxic T lymphocyte response to Hepatitis B virus surface antigen. *Hum. Gene Ther. 6*:1447.

26. **Schirmbeck, R., W. Böhm, K. Ando, F. V. Chisari, and J. Reimann**. 1995. Nucleic acid vaccination primes hepatitis B surface antigen-specific cytotoxic T lymphocytes in nonresponder mice. *J. Virol. 69*:5929.

27. **Böhm, W., A. Kuhröber, T. Paier, T. Mertens, J. Reimann, and R. Schirmbeck**. 1996. DNA vector constructs that prime hepatitis B surface antigen-specific cytotoxic T lymphocytes and antibody responses in mice after intramuscular injection. *J. Jmmunol. Methods In press*:

28. **Szikora, J.-P., A. van Pel, V. Brichard, M. Andre, N. van Baren, P. Henry, E. de Plaen, and T. Boon**. 1990. Structure of the gene of tum- transplantation antigen P35B:presence of a point mutation in the antigenic allele. *EMBO J. 9*:1041.

29. **Van den Eynde, B., B. Lethe, A. van Pel, E. de Plaen, and T. Boon**. 1991. The gene coding for a major tumor rejection antigen of tumor P815 is identical to the normal gene of syngeneic DBA/2 mice. *J. Exp. Med. 173*:1373.

30. **Chomez, P., E. de Plaen, A. van Pel, C. de Smet, J. P. Szikora, C. Lurquin, A. M. Lebacq Verheyden, and T. Boon**. 1992. Efficient expression of tum- antigen P91A by transfected subgenic fragments. *Immunogenetics 35*:241.

31. **Dyson, P. J., C. de Smet, A. M. Knight, D. Simon Chazottes, J. L. Guenet, and T. Boon**. 1992. Mapping of the genes encoding tum- transplantation antigens P91A, P35B, and P198. *Immunogenetics 35*:316.

32. **Lethe, B., B. Van den Eynde, A. van Pel, G. Corradin, and T. Boon**. 1992. Mouse tumor rejection antigens P815A and P815B: two epitopes carried by a single peptide. *Eur. J. Immunol. 22*:2283.

33. **Szikora, J. P., A. van Pel, and T. Boon**. 1993. Tum- mutation P35B generates the MHC-binding site of a new antigenic peptide. *Immunogenetics 37*:135.

34. **Brichard, V. G., G. Warnier, A. van Pel, G. Morlighem, S. Lucas, and T. Boon**. 1995. Individual differences in the orientation of the cytolytic T cell response against mouse tumor P815. *Eur. J. Immunol. 25*:664.

35. **Grohmann, U., R. Bianchi, M. C. Fioretti, F. Fallarino, L. Binaglia, C. Uyttenhove, A. van Pel, T. Boon, and P. Puccetti**. 1995. CD8+ cell activation to a major mastocytoma rejection antigen, P815AB: requirement for tumor helper peptides in priming for skin test reactivity to a P815AB-related peptide. *Eur. J. Immunol. 25*:2797.

36. **Schirmbeck, R., K. Melber, and J. Reimann**. 1995. Hepatitis B virus surface particles are processed in a novel endosomal pathway for MHC class I-restricted epitope presentation. *Eur. J. Immunol. 25*:1063.

37. **Karasuyama, H. and F. Melchers**. 1988. Establishment of mouse cell lines which constituticely secrete large quantities of interleukin 2, 3, 4 and 5, using modified cDNA expression vectors. *Eur. J. Immunol. 18*:97.

38. **Davis, H. L., B. A. Demeneix, B. Quantin, J. Coulombe, and R. G. Whalen**. 1993. Plasmid DNA is superior to viral vectors for direct gene transfer into adult mouse skeletal muscle. *Hum. Gene Ther. 4*:733.

39. **Davis, H. L., M. L. Michel, and R. G. Whalen**. 1993. DNA-based immunization induces continuous secretion of hepatitis B surface antigen and high levels of circulating antibody. *Hum. Mol. Genet. 2*:1847.

40. **Cobbold, S. P., A. Jayasuriya, A. Nash, T. D. Prospero, and H. Waldmann**. 1984. Therapy with monoclonal antibodies by elimination of T-cell subsets in vivo. *Nature 312*:548.

41. **Levraud, J.-P., C. Pannetier, P. Langlade-Demoyen, V. Brichard, and P. Kourilsky**. 1996. Recurrent T cell receptor rearrangements in the cytotoxic T lymphocyte response *in vivo* against the P815 murine tumor. *J. Exp. Med. 183*:430.

42. **Plata, F., P. Langlade-Demoyen, J.-P. Abastado, T. Berbar, and P. Kourilsky**. 1987. Retrovirus antigens recognized by cytolytic T lymphocytes activate tumor rejection in vivo. *Cell 48*:231.

43. **Kast, W. M., R. Offringa, P. J. Peters, A. C. Voordouw, R. H. Meloen, A. J. van der Eb, and C. J. M. Melief**. 1989. Eradication of adenovirus E1-induced tumors by E1A-specific cytotoxic T lymphocytes. *Cell 59*:603.

44. **Chen, L., M. T. Mizuno, M. C. Singhal, S. L. Hu, D. A. Galloway, I. Hellström, and K. E. Hellström**. 1992. Induction of cytotoxic T lymphocytes specific for a syngeneic tumor expressing the E6 oncoprotein of human papillomavirus type 16. *J. Jmmunol. 148*:2617.

45. **Chen, L., P. McGowan, S. Ashe, J. V. Johnston, I. Hellström, and K. E. Hellström**. 1994. B7-1/CD80-transduced tumor cells elicit better systemic immunity than wild-type tumor cells admixed with Corynebacterium parvum. *Cancer Res. 54*:5420.

46. **Celluzzi, C. M., J. I. Mayordomo, W. J. Storkus, M. T. Lotze, and L. D. Falo,Jr**. 1996. Peptide-pulsed dendritic cells induce antigen-specific CTL-mediated protective tumor immunity [see comments]. *J. Exp. Med. 183*:283.

47. **Paglia, P., C. Chiodoni, M. Rodolfo, and M. P. Colombo**. 1996. Murine dendritic cells loaded in vitro with soluble protein prime cytotoxic T lymphocytes against tumor antigen in vivo [see comments]. *J. Exp. Med. 183*:317.

48. **Nabel, G. J., E. G. Nabel, Z. Y. Yang, B. A. Fox, G. E. Plautz, X. Gao, L. Huang, S. Shu, D. Gordon, and A. E. Chang**. 1993. Direct gene transfer with DNA-liposome complexes in melanoma: expression, biologic activity, and lack of toxicity in humans. *Proc. Natl. Acad. Sci. in. S. A. 90*:11307.

49. **Plautz, G. E., Z. Y. Yang, B. Y. Wu, X. Gao, L. Huang, and G. J. Nabel**. 1993. Immunotherapy of malignancy by in vivo gene transfer into tumors [see comments]. *Proc. Natl. Acad. Sci. in. S. A. 90*:4645.

50. **Nabel, G. J., A. E. Chang, E. G. Nabel, G. E. Plantz, W. Ensminger, B. A. Fox, P. Felgner, S. Shu, and K. Cho**. 1994. Immunotherapy for cancer by direct gene transfer into tumors. *Hum. Gene Ther. 5*:57.

51. **Huang, A. Y., P. Golumbek, M. Ahmadzadeh, E. Jaffee, D. Pardoll, and H. Levitsky**. 1994. Role of bone marrow-derived cells in presenting MHC class I-restricted tumor antigens. *Science 264*:961.

52. **Raposo, G., H. W. Nijman, W. Stoorvogel, R. Leijendekker, C. V. Harding, C. J. Melief, and H. J. Geuze**. 1996. B lymphocytes secrete antigen-presenting vesicles. *J. Exp. Med. 183*:1161.

53. **Casciola-Rosen, L., A. Rosen, M. Petri, and M. Schissel**. 1996. Surface blebs on apoptotic cells are sites of enhanced procoagulant activity: implications for coagulation events and antigenic spread in systemic lupus erythematosus. *Proc. Natl. Acad. Sci. U. S. A. 93*:1624.

54. **Kündig, T. M., M. F. Bachmann, C. Dipaolo, J. J. L. Simard, M. Battegay, H. Lother, A. Gessner, K. Kuhlke, P. S. Ohashi, H. Hengartner, and R. M. Zinkernagel**. 1995. Fibroblasts as efficient antigen-presenting cells in lymphoid organs. *Science 268*:1343.

55. **Sercarz, E. E., P. V. Lehmann, A. Ametani, G. Benichou, A. Miller, and K. Moudgil**. 1993. Dominance and crypticity of T cell antigenic determinants. *Annu. Rev. Immunol. 11*:729.

56. **Johnston, J. V., A. R. Malacko, M. T. Mizuno, P. McGowan, I. Hellström, K. E. Hellström, H. Marquardt, and L. Chen**. 1996. B7-CD28 costimulation unveils the hierarchy of tumor epitopes recognized by major histocompatibility complex class I-restricted CD8[+] cytolytic T lymphocytes. *J. Exp. Med. 183*:791.

**Claims**

1. A polycistronic expression plasmid encoding one or more viral antigens suitable for

   (i) DNA based immunization of a cancer-bearing patient and/or (ii) transfection in vitro and/or in vivo of tumor cells of said patient.

2. A polycistronic expression plasmid according to claim 1 encoding in addition one or more cytokines.

3. A polycistronic expression plasmid according to claim 1 or 2, characterized in that it encodes an antigen against which an anti-viral activity of the cancer-bearing patient is directed, which plasmid is to be injected into tumor cells of said patient.

4. A polycistronic expression plasmid according to any of the preceding claims, encoding one or more antigens of common viral pathogens, especially measles, rubella, varicella, herpes virus and hepatitis B.

5. Tumor cells of a cancer-bearing patient, wherein the tumor cells are transfected in vitro with a polycistronic expression plasmid according to any of the preceding claims for injection into said patient.

6. A process for the production of a polycistronic expression plasmid according to any of the preceding claims, characterized by the following steps:

   (a) in vitro screening for a specific cytolytic T cell reactivity in peripheral blood of a cancer-bearing patient, wherein said reactivity is directed against one or more viral antigens,
   (b) cloning one or more of said viral antigens and, optionally, one or more cytokines into a polycistronic expression vector.

7. A process according to claim 6, characterized in that the viral antigens are selected from the group of measles, rubella, varicella, herpes virus and hepatites B.

8. Use of a polycistronic expression vector obtained according to the process according to claim 6 or 7 for an immunotherapy of cancer, characterized by

   (a) injecting in vivo the polycistronic expression vector into tumor cells of the cancer bearing patient; or
   (b) isolating tumor cells from said patient, transfecting them in vitro with said polycistronic expression vector, and re-injecting the resulting genetically engineered tumor cells back into the patient; or
   (c) transfecting in vitro tumor cells having been isolated from said cancer-bearing patient, with said polycistronic expression vector for re-injecting the resulting genetically engineered tumor cells back into the patient.

Figure 1

Subcutaneous growth of transplanted non-transfectected and transfected tumor cells

(A) P815 cells

(B) P815/S transfectants

Figure 2

Transfer of P815/S cells into P815 tumors growing
subcutaneously in non-immune or immune, syngeneic hosts

(A) non-immune host

(B) immune host

days post-transplantation

days post-transplantation

Figure 3

EP 0 805 207 A1

Tumor (P815)-specific cytotoxic T lymphocyte are generated in the
HBsAg-mediated rejection process

**European Patent Office**

## PARTIAL EUROPEAN SEARCH REPORT

**Application Number**

which under Rule 45 of the European Patent Convention EP 96 10 6935
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X,D | GENETIC ENGINEERING NEWS, 1 March 1996, pages 14-16, XP002012721 DIFMOND P.F: "Therion combines technologies to produce vaccines and immunotherapeutics" | 1-5 | C12N15/62 C07K14/005 C07K14/195 C07K14/37 C07K14/44 C07K14/47 C07K14/52 A61K39/295 |
| Y | * the whole document * | 6 | |
| X | EP-A-0 406 857 (TAKEDA CHEMICAL INDUSTRIES LTD) 9 January 1991 * page 4, line 50 - page 5, line 3; claims 1-35 * | 1-4 | |
| X | US-A-4 859 465 (RUTTER WILLIAM J) 22 August 1989 * claims 1-4; example 2 * | 1-4 | |
| A | EP-A-0 154 576 (INTERFERON SCIENCES INC) 11 September 1985 * abstract; claims 1,2,6 * | 1-4 | |
|  | -/-- | | |

**TECHNICAL FIELDS SEARCHED (Int.Cl.6)**

C07K
C12N

### INCOMPLETE SEARCH

The Search Division considers that the present European patent application does not comply with
the provisions of the European Patent Convention to such an extent that it is not possible to carry
out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 3 October 1996 | Gurdjian, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent
Office

**PARTIAL EUROPEAN SEARCH REPORT**

Application Number

EP 96 10 6935

| | DOCUMENTS CONSIDERED TO BE RELEVANT | | CLASSIFICATION OF THE APPLICATION (Int.Cl.6) |
|---|---|---|---|
| Category | Citation of document with indication, where appropriate, of relevant passages | Relevant to claim | |
| Y | WO-A-95 28479 (KLINIKUM DER ALBERT LUDWIGS UN ;KANZ LOTHAR (DE); BRUGGER WOLFRAM) 26 October 1995 <br> * page 4, paragraph 5 - page 5, paragraph 1; example 7 * <br> ----- | 6 | |
| | | | **TECHNICAL FIELDS SEARCHED** (Int.Cl.6) |

EPO FORM 1503 03.82 (P04C10)

EP 96 10 6935

-C-

Remark: Although claim 8
is directed to a method of
treatment of the human/animal
body (Art. 52(4) EPC) the search
has been carried out and based on
the alleged effects of the
compound/composition